(19) Europäisches Patentamt
European Patent Office
Office européen des brevets

(11) EP 3 789 377 A1

(12) EUROPEAN PATENT APPLICATION

(43) Date of publication:
**10.03.2021 Bulletin 2021/10**

(21) Application number: **19382748.2**

(22) Date of filing: **03.09.2019**

(51) Int Cl.:
***C07C 45/49*** *(2006.01)* ***C07C 49/597*** *(2006.01)*
***C07C 69/757*** *(2006.01)* ***C07F 7/08*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Fundación Universitaria San Pablo-Ceu**
  **28040 Madrid (ES)**
- **Csflowchem, S.L.**
  **28050 Madrid (ES)**

(72) Inventors:
- **Pérez Castells, Javier**
  **28043 MADRID (ES)**
- **Domínguez Martín, Gema**
  **28043 MADRID (ES)**
- **García Lacuna, Jorge**
  **28039 MADRID (ES)**
- **Blanco Urgoiti, Jaime**
  **28050 MADRID (ES)**

(74) Representative: **ZBM Patents - Zea, Barlocci & Markvardsen**
**Rambla Catalunya, 123**
**08008 Barcelona (ES)**

# (54) A CATALYTICALLY CONTINUOUS FLOW PAUSON-KHAND REACTION WITHOUT CARBON MONOXIDE GAS

(57) The present invention relates to a continuous flow process for the preparation of a cyclopentenone ring-containing compound which comprises: reacting an alkyne-moiety with an alkene-moiety in the presence of a catalytically effective amount of one or more metal carbonyl compounds, in the absence of exogenous carbon monoxide gas. The invention also relates to a process for the preparation of cyclopentanol ring-containing compound which comprises performing the continuous flow process for the preparation of a cyclopentenone ring-containing compound, and particularly for the preparation of treprostinil.

EP 3 789 377 A1

## Description

The invention relates to a continuous flow Pauson-Khand reaction. In particular, to a catalytically continuous flow Pauson-Khand reaction without carbon monoxide gas. It also related to a process for the preparation of a cyclopentanol ring containing compound comprising performing the catalytically continuous flow Pauson-Khand reaction without carbon monoxide gas.

## Background Art

The Pauson-Khand reaction (also named PKR or PK-type reaction) is a chemical reaction described as a [2+2+1] cycloaddition between an alkyne moiety, an alkene moiety and carbon monoxide in the presence of a metal carbonyl compound to form a $\alpha,\beta$-cyclopentenone as it is shown in the scheme 1.

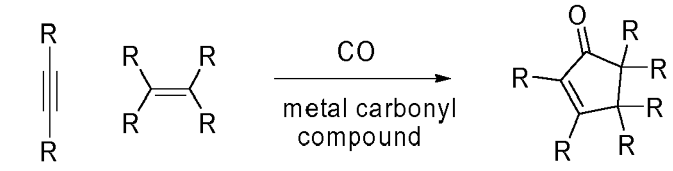


alkyne alkene alpha,beta-cyclopentenone

Scheme 1

Very few reactions produce in a single step an increase in molecular complexity such as the Pauson-Khand (RPK) reaction. This reaction was originally performed under stoichiometric amounts of the reagents, but catalytic versions of the Pauson-Khand reaction has been also disclosed in the state of the art. In the stoichiometric version, one of the most used metal carbonyl compounds is a cobalt carbonyl compound (such as for example octacarbonyl), but alternative metal carbonyl compounds using ruthenium or molybdenum as a metal have also been disclosed in the state of the art.

Although the Pauson-Khand reaction produces useful products, it suffers from some drawbacks. One of these drawbacks is that the metal carbonyl compound (commonly a cobalt carbonyl compound) and its analogues are volatile, toxic, and unstable to loss carbon monoxide and to aerial oxidation. Accordingly, these metal carbonyl reagents pose hazards in storage, use, disposal, and product purification. In addition, generally, the resulting cyclopentenone ring containing product may retain metal impurities, especially when used stoichiometrically. Furthermore, in order to accelerate the reaction, the addition into the reaction media of promoters such as cyclohexylamine, sulphides, phosphates or amine N-oxides has been disclosed in the state of the art. Unfortunately, even the use of these additives as promoting agents, the conversion and speed of the Pauson-Khand reaction is not completely restored. Therefore, the use of catalytic amounts of the metal carbonyl compound are recommended.

However, when catalytic amount of the metal carbonyl compound is used, the reaction conversion, speed and yield of the Pauson-Khand reaction is compromised. In order to accelerate the reaction, the use of chemical additives has been suggested. However, these additives do not restore the conversion and speed of the Pauson-Khand reaction as in the use of the stoichiometric reaction. Furthermore, focus on green chemistry, it is also unthinkable for a reaction to be used on an industrial level, if it involves metal complexes, and it is not efficiently catalytic.

Another of the drawbacks of the catalytic Pauson-Khand reaction is the use of carbon monoxide gas. Carbon monoxide (CO) is an extremely poisonous gas. Therefore, it is important to take proper safety precautions when using it, especially at high pressure. The reactions in which this gas is used pose a very high risk especially in conventional laboratories or when the use of a large quantity is required. Handling such a highly toxic gas is discouraging for many groups and is a major problem in escalating the reaction at industrial scale. Historically the first achievement for the reduction of the carbon monoxide was to decrease the necessary CO pressure. After these first studies, several other alternatives have been disclosed, which involve the use of additives for forming a more stable metal carbonyl compound that could re-enter a catalytic cycle before decomposing and, thus reducing the amount of CO needed. In particular, the most recent approach for avoiding the use of carbon monoxide in the Pauson-Khand reaction has been to use $CO_2$ in a reducing medium to produce CO in situ. In a recent study a mesoporous graphite carbon nitride (mpg-C3N4) was used as a catalyst that allowed direct activation of $CO_2$. In the reaction benzene was used which was oxidized to phenol while the CO formed was used for a later RPK. In another study, $CO_2$ was efficiently reduced with triphenylsilane by using CsF in a sealed two-chamber reactor. The system was used for various reactions such as palladium catalysed.

Although the use of a reduced amount of carbon monoxide gas is advantageous, it would be desirable to avoid completely the handling of this dangerous and toxic gas and reducing the use of further additives. Therefore, from what

is known in the art it is derived that there is still the need of providing an efficient catalytically Pauson-Khand reaction without the use of carbon monoxide gas.

## Summary of Invention

The inventors of the present invention have surprisingly provided an industrial scaling up and efficient Pauson-Khand reaction for the preparation of cyclopentenone ring-containing compound which comprises reacting an alkyne moiety with a alkene moiety in a continuous flow with the use of a catalytically effective amount of the metal carbonyl compound and also in the absence of "exogenous" addition of carbon monoxide gas. In particular, the inventors have found that the Pauson-Khand of the present invention allows preparing cyclopentenone ring-containing compounds with a high conversion, high chemical yield and high diastereoselectivity (if possible). Furthermore, the reaction process of the present invention is safer than the processes disclosed in the state of the art because of the absence of carbon monoxide gas and for the reduction of the handling of hazardous compounds (i.e. reagents, starting materials and by-products). It is also environmentally friendly due to the use of a catalytically amount of the metal carbonyl compound producing lower amounts of hazardous wastes.

Moreover, the Pauson-Khand of the present invention is very versatile, since it can be performed with a large number of the starting materials (i.e. alkyne-containing compounds plus alkene-containing compounds and alkyne-alkene containing compounds), even those which have steric hindrance as it is shown in the experimental data. Thus, the Pauson-Khand of the invention can be performed by means of an intramolecular or intermolecular reaction. Finally, as the reaction of the present is performed in a continuous flow, it is advantageous because it is faster than that performed in batch. In fact, the flow of the Pauson-Khand of the invention can be gradually increased in a much easier way than the corresponding batch reaction, simply by leaving the process longer or by using larger flows and larger capacity reactors accordingly.

The inventors have also surprisingly found that the Pauson-Khand reaction of the present invention is also useful for the preparation of cyclopentanol ring-containing compound. In particular, this process comprises firstly, preparing the cyclopentenone ring-containing compound following the process of the present invention followed by its conversion into cyclopentanol ring-containing compound.

The process for the preparation of the cyclopentanol ring-containing compound is also efficient because also has a high conversion and high chemical yield. In case that the cyclopentanol ring-containing compound has diastereoisomers, the process has a high diastereoselectivity. Particularly, this process allows preparing efficiently pharmaceutically active ingredients having a cyclopentanol ring. Particularly, the process for the preparation of the cyclopentanol ring-containing compound allows manufacturing the pharmaceutical active ingredient treprostinil of formula (1) as defined below.

Thus, the first aspect of the invention relates to a continuous flow process for the preparation of a cyclopentenone ring-containing compound which comprises: a continuous flow process for the preparation of a cyclopentenone ring-containing compound which comprises: reacting an alkyne-moiety with an alkene-moiety in the presence of a catalytically effective amount of one or more metal carbonyl compounds, in the absence of exogenous carbon monoxide gas; wherein: when the process is an intermolecular process, the process comprises reacting the alkyne-moiety of an alkyne-containing compound with the alkene-moiety of an alkene-containing compound; and when the process is an intramolecular process, the process comprises reacting the alkyne-moiety with the alkene-moiety of an alkyne-alkene containing compound.

In particular, the invention relates to a process for the preparation of the cyclopentenone ring-containing compound of formula (3) as defined below from the alkyne-alkene containing compound of formula (2) under the reaction conditions of the process of the first aspect of the invention.

The second aspect of the invention relates to a process for the preparation of cyclopentanol ring-containing compound which comprises: a process for the preparation of cyclopentanol ring-containing compound which comprises: a) performing the continuous flow process as defined in the first aspect of the invention to yield a cyclopentenone ring-containing compound; and b) submitting the cyclopentenone ring-containing compound under such reaction conditions to convert the cyclopentenone ring-containing compound into the cyclopentanol ring-containing compound.

In particular, the invention relates to a process for the preparation of the cyclopentanol ring-containing compound of formula (1) as defined below from the cyclopentenone ring-containing compound of formula (3) under the reaction conditions of the process of the second aspect of the invention.

The third aspect of the invention relates to the compounds of formula (2), (3) and (4) as defined below.

## Detailed description of the invention

All terms as used herein in this application, unless otherwise stated, shall be understood in their ordinary meaning as known in the art. Other more specific definitions terms as used in the present application are as set forth below and are intended to apply uniformly throughout the specification and claims unless an otherwise expressly set

out definition provides a broader definition.

For the purposes of the present invention, any ranges given include both the lower and the upper end-points of the range. Ranges given, such as temperatures, times, weights, and the like, should be considered approximate, unless specifically stated.

The term "alkyl" refers to a saturated straight, or branched hydrocarbon chain which contains the number of carbon atoms specified in the description or claims. Examples include, among others, the group methyl, ethyl, propyl, isopropyl, butyl, isobutyl, sec-butyl, and tert-butyl.

The term "cycloalkyl" refers to a saturated straight, or branched hydrocarbon ring, which contains the number of carbon atoms specified in the description or claims, that may be substituted at any available ring position.

The term "cycloheteroalkyl" refers to a saturated straight, or branched hydrocarbon ring, containing from one to four heteroatoms independently selected from O, S and N, which contains the number of carbon atoms specified in the description or claims, that may be substituted at any available ring position.

As it is mentioned above, the first aspect of the invention relates to a continuous flow process for the preparation of a cyclopentenone ring-containing compound (also named in the present invention as Pauson-Khand reaction). As used herein, the terms "continuous process" or "continuous flow process" have the same meaning and they are used interchangeable. They refer to a process having constant input and output used to manufacture, produce, or process materials without interruption. In continuous production mode the materials being processed are continuously in motion, undergoing chemical reactions or subject to mechanical or heat treatment. Continuous processing is contrasted with batch production. The terms "batch production" or "batch process" are a technique used in manufacturing, in which the object in question is created stage by stage over a series of workstations, and different batches of products are made.

The process of the invention comprises reacting an alkyne-moiety with an alkene-moiety. In particular, wherein the process is an intermolecular process, then the process comprises reacting the alkyne-moiety of an alkyne-containing compound with the alkene-moiety of an alkene-containing compound; and wherein the process is an intramolecular process, then the process comprises reacting the alkyne-moiety with the alkene-moiety of an alkyne-alkene containing compound. The term "alkyne-moiety" refers to a carbon-carbon triple bond. Thereby, a "alkyne-containing compound" refers to an organic compound containing at least one carbon-carbon triple bond. The term "alkene-moiety" refers to a carbon-carbon double bond. Thereby, a "alkene-containing compound" refers to an organic compound containing at least one carbon-carbon double bond. The term "alkyne-alkene containing compound" refers to an organic compound containing at least one carbon-carbon double bond and at least one carbon-carbon triple bond. In an embodiment, the process is an intermolecular process and comprises reacting the alkyne-moiety of an alkyne-containing compound with the alkene-moiety of an alkene-containing compound. In an embodiment, the process is an intramolecular process and comprises reacting the alkyne-moiety with the alkene-moiety of an alkyne-alkene containing compound.

The process of the invention is performed in the presence of a catalytically effective amount of one or more metal carbonyl compounds. For the purpose of the invention, the term "catalytically effective amount" refers to the fact that the amount of the metal carbonyl compound is much smaller than the stoichiometric amount but enough for catalysing the reaction. In an embodiment, the process is performed in the presence of a catalytically effective amount of the metal carbonyl compound from 10 to 40 mol% of the alkyne containing compound or alternatively of the alkyne-alkene containing compound. In an embodiment, the process is performed in the presence of a catalytically effective amount of the metal carbonyl compound from 10 to 30 mol% of the alkyne containing compound or alternatively of the alkyne-alkene containing compound. In an embodiment, the process is performed in the presence of a catalytically effective amount of the metal carbonyl compound from 15 to 20 mol% of the alkyne containing compound or alternatively of the alkyne-alkene containing compound. The terms "mol%", "molar%" and "mole percent" have the same meaning and are used interchangeable. Mole percent is the percentage that the moles of a component (metal carbonyl compound) are in relation to the moles of another particular component. For example, in the present invention, the molar % is the percentage of moles of the metal carbonyl compound in relation to the moles of the alkyne containing compound or alternatively of the alkyne-alkene containing compound.

In an embodiment, the metal carbonyl compound comprises a metal being a transition metal. In an embodiment, the metal carbonyl compound comprises a metal selected from the group consisting of cobalt, rhodium, iridium, tungsten, molybdenum, titanium, nickel, iron and ruthenium. In an embodiment, the metal carbonyl compound comprises cobalt as a metal. In an embodiment, the metal carbonyl compound is dicobalt octacarbonyl.

The Pauson-Khand of the first aspect of the invention is performed in the absence of exogenous carbon monoxide gas. The term "exogenous" refers to the fact that the gas has an external cause or origin, and therefore the gas is externally added to the reaction media. Thus, the process of the invention involves an internal transfer of the carbon monoxide from the metal carbonyl compound to the cyclopentenone ring-containing compound.

The Pauson-Khand of the first aspect of the invention is an efficient process having a high conversion and high yield even that the process is performed using a catalytically effective amount of the metal carbonyl compound and in the absence of exogeneous carbon monoxide gas. In an embodiment, the Pauson-Khand of the invention has a reaction conversion equal to or higher than 90%; particularly equal to or higher than 95%; more particularly equal to or higher

than 99%. The reaction conversion can be calculated by any method known in the state of the art. In the case of the present invention the reaction conversion is calculated by high performance liquid chromatography/Nuclear Magnetic Resonance (HPLC/$^1$H-NMR). In an embodiment, the Pauson-Khand of the invention has a chemical yield of the cyclopentenone ring-containing compound equal to or higher than 60%; particularly equal to or higher than 65%; more particularly equal to or higher than 70%. The chemical yield can be calculated by any method known in the state of the art. In the case of the present invention the chemical yield is calculated by HPLC. In an embodiment, the Pauson-Khand of the invention has a reaction conversion equal to or higher than 90% and a chemical yield of the cyclopentenone ring-containing compound equal to or higher than 60%. In an embodiment, the Pauson-Khand of the invention has a reaction conversion equal to or higher than 95% and a chemical yield of the cyclopentenone ring-containing compound equal to or higher than 65%. In an embodiment, the Pauson-Khand of the invention has a reaction conversion equal to or higher than 99% and a chemical yield of the cyclopentenone ring-containing compound equal to or higher than 70%.

When the cyclopentenone ring-containing compound has one or more stereogenic carbon atom, then the Pauson Khand of the invention allows obtaining it diastereoselectivity enriched. The terms "stereogenic carbon atom", "chiral carbon atom" or "asymmetric carbon atom" have the same meaning, and are used interchangeably. A chiral carbon atom refers to a carbon atom to which four different atoms or groups of atoms are attached. The term "diastereoselectively enriched" refers to a chiral non-racemic compound, that is, a compound which has more of one diastereomer than another diastereomer. The degree of enrichment of one diastereomer is measured by the diastereomeric excess (*de*). The "diastereomeric excess" or "*de*" is a measure of the excess of one diastereomer over an equimolar mixture of diastereomers of a chiral compound, which is commonly expressed as a percentage. Diastereomeric excess is defined as the absolute difference between the mole fractions of each diastereomer. If the moles of each diastereomer are known, the percent diastereomeric excess can be determined by the following formula:

$$de = (A-B)/(A+B) * 100$$

being A and B the molar fraction or the number of moles of diastereomer A and diastereomer B. In an embodiment, the Pauson-Khand reaction of the invention allows obtaining a cyclopentenone ring-containing compound having a diastereomeric excess equal to or higher than 95%; particularly equal to or higher than 97%.

In an embodiment, the Pauson-Khand of the invention has a reaction conversion equal to or higher than 90%, a chemical yield of the cyclopentenone ring-containing compound equal to or higher than 60%, and a diastereomeric excess equal to or higher than 95%.

In an embodiment, the Pauson-Khand of the invention is performed at a temperature from 100 to 180°C; particularly from 110 to 170°C. In an embodiment, the Pauson-Khand of the invention is performed at a pressure from 1000 kPa to 3000 kPa; particularly from 1500 kPa to 2500 kPa; more particularly from 1500 kPa to 20 kPa. It is advantageous because the process of the invention is performed at mild reaction conditions.

In an embodiment, the Pauson-Khand of the invention is performed in the presence of an organic solvent. In an embodiment, the Pauson-Khand of the invention is performed in the presence of an organic solvent selected from the group consisting of ($C_1$-$C_{16}$) hydrocarbon, ($C_1$-$C_6$)alcohol, ($C_4$-$C_6$)alkyl-O-($C_4$-$C_6$)alkyl and a mixture thereof. In an embodiment, the Pauson-Khand of the invention is performed in the presence of an organic solvent selected from the group consisting of toluene, xylene, naphthalene, isopropanol, ethanol, tert-butanol, tetrahydrofuran, 2-methyltetrahydrofuran, dioxane, and a mixture thereof. In an embodiment, the Pauson-Khand of the invention is performed in the presence of the organic hydrocarbon solvent toluene.

In a particular embodiment, the continuous flow process is an intramolecular process and the alkyne-alkene containing compound is a compound of formula (2)

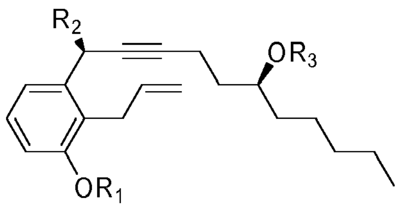

(2)

and the cyclopentenone ring-containing compound is a compound of formula (3)

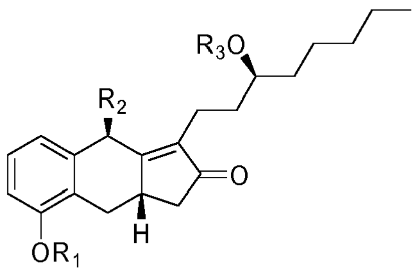

(3)

wherein:

- $R_1$ is PG-O-CO-$CH_2$- or a group convertible into HO-CO-$CH_2$-;
- $R_2$ is a HO-, PG-O- or a group convertible into H;
- $R_3$ is H- or PG-;
- PG is a hydroxy protective group selected from ($C_1$-$C_6$)alkyl-, ($C_1$-$C_6$)alkyl-CO-O-, ($C_1$-$C_{16}$)alkyl-O-, ($C_1$-$C_{16}$)alkyl-CO-, (($C_1$-$C_{16}$)alkylh)$_3$-Si-, ($C_1$-$C_6$)-phenethyl-, ($C_1$-$C_6$)-phenethyl-$CH_2$-,
- and wherein, each one of $R_1$, $R_2$ and $R_3$ is stable under the reaction conditions and is a de-protected form or an intermediate protected form of it that can be transformed after that into said de-protected form in the needed measure.

The term "protective group" (PG) as used herein refers to a grouping of atoms that when attached to a reactive group in a molecule masks, reduces or prevents that reactivity. The term "hydroxy protective group" refers to the protective group used to protect a hydroxyl group of the compounds disclosed in the present invention. This term encompasses all the protective groups defined in e.g. T. W. Green and P. G. M. Wuts, Protective Groups in Organic Chemistry (Wiley, 3rd ed. 1999, Chapter 2, pp. 17-200).

All the embodiments defined above for the continuous flow process of the first aspect of the invention also applies for the preparation of the cyclopentenone ring-containing compound of formula (3) from the alkyne-alkene containing compound of formula (2).

In an embodiment, the continuous flow process is an intramolecular process and the alkyne-alkene containing compound is the compound of formula (2) as defined above and the cyclopentenone ring-containing compound is the compound of formula (3) as defined above, wherein:

- $R_1$ is PG-O-CO-$CH_2$- or a group convertible into HO-CO-$CH_2$-;
- $R_2$ is a HO-, PG-O- or a group convertible into H;
- $R_3$ is H- or PG-;
- PG is a hydroxy protective group selected from the group consisting of a silane-containing protective group of formula (($C_1$-$C_{16}$)alkyl)$_3$-Si-.

In an embodiment, the continuous flow process is an intramolecular process and the alkyne-alkene containing compound is the compound of formula (2) as defined above and the cyclopentenone ring-containing compound is the compound of formula (3) as defined above, wherein:

- $R_1$ is PG-O-CO-$CH_2$- or a group convertible into HO-CO-$CH_2$-;
- $R_2$ is a HO-, PG-O- or a group convertible into H;
- $R_3$ is H- or PG-;
- PG is a hydroxy protective group selected from the group consisting of a silane-containing protective group of formula (($C_1$-$C_{16}$)alkyl)$_3$-Si- selected from the group consisting of trimethylsilyl, *tert*-butyldimethylsilyl, *tert*-butyldiphenylsilyl, triisopropylsilyl, triethylsilyl, diethylisopropylsilyl and isopropyldimethylsilyl

In an embodiment, the continuous flow process is an intramolecular process and the alkyne-alkene containing compound is the compound of formula (2) as defined above and the cyclopentenone ring-containing compound is the compound of formula (3) as defined above, wherein:

- $R_1$ is PG-O-CO-$CH_2$- and PG is ($C_1$-$C_6$)alkyl-;
- $R_2$ is PG-O- and PG is a hydroxy protective group selected from the group consisting of a silane-containing protective

group of formula (($C_1$-$C_{16}$)alkyl)$_3$-Si- selected from the group consisting of trimethylsilyl, *tert*-butyldimethylsilyl, *tert*-butyldiphenylsilyl, triisopropylsilyl, triethylsilyl, diethylisopropylsilyl and isopropyldimethylsilyl
$R_3$ is PG-O and PG is a hydroxy protective group selected from the group consisting of a silane-containing protective group of formula (($C_1$-$C_{16}$)alkyl)$_3$-Si- selected from the group consisting of trimethylsilyl, *tert*-butyldimethylsilyl, *tert*-butyldiphenylsilyl, triisopropylsilyl, triethylsilyl, diethylisopropylsilyl and isopropyldimethylsilyl

In an embodiment, the continuous flow process is an intramolecular process and the alkyne-alkene containing compound is the compound of formula (2) as defined above and the cyclopentenone ring-containing compound is the compound of formula (3) as defined above, wherein:

$R_1$ is PG-O-CO-$CH_2$- and PG is $(CH_3)_3C$-;
$R_2$ is PG-O and PG is $(CH_3)_3C$-Si(H)-$(CH_3)_2$-; and
$R_3$ is PG- and PG is $(CH_3)C$-Si(phenyl)$_2$-.

The second aspect of the invention relates to a process for the preparation of cyclopentanol ring-containing compound which comprises:

a) performing the continuous flow process as defined in the first aspect of the invention to yield a cyclopentenone ring-containing compound; and
b) submitting the cyclopentenone ring-containing compound under such reaction conditions to convert the cyclopentenone ring-containing compound into the cyclopentanol ring-containing compound.

All the embodiments disclosed above for the Pauson-Khand reaction of the first aspect of the invention also apply to step a) of the process of the second aspect of the invention.
In an embodiment, step (b) of the process for the preparation of cyclopentanol ring-containing compound comprises:

b1) converting the cyclopentenone ring-containing compound into a cyclopentanone ring-containing compound; and
b2) converting the cyclopentanone ring-containing compound into the cyclopentanol ring-containing compound.

In an embodiment, step b1) of the process comprises reducing the double bond of the cyclopentenone to cyclopentanone with hydrogen in the presence of a catalyst and in the sinus of an organic solvent. In an embodiment, step b1) of the process of the invention comprises reducing the double bond of the cyclopentenone with hydrogen in the presence of Pd/C and an organic solvent.
In an embodiment, step b1) of the process of the invention comprises reducing the double bond of the cyclopentenone with hydrogen in the presence of Pd/C and ($C_1$-$C_6$)alcohol such as ethanol as a solvent.
In an embodiment, step b1) of the process of the invention comprises reducing the double bond of the cyclopentenone with hydrogen at a hydrogen pressure from 0.1 MPa to 0.5 MPa; particularly from 0.2 MPa to 0.3 MPa.
In an embodiment, step b1) of the process of the invention comprises reducing the double bond of the cyclopentenone with hydrogen in the presence of a catalyst, particularly Pd/C, in an amount from 10% (w/w) to 30% (w/w).
The terms "percentage (%) by weight" and "percentage (%) w/w" have the same meaning and they are used interchangeable. They refer to the percentage of a compound in relation to another compound. For example, the amount of the catalyst in relation to the amount of the cyclopentenone.
In an embodiment, step b1) of the process of the invention is performed at a temperature from 0°C to 40°C; particularly, from 15°C to 25°C.
In an embodiment, step b1) of the process of the invention is performed at a concentration of the cyclopentenone from 0.05M to 0.4M; particularly from 0.1 M to 0.25M.
The term "concentration" in chemistry refers to the number of moles of a solute divided by the number of litres of solution(mol/L) expressed as Molarity (M).
In an embodiment, step b1) of the process of the invention is performed in the presence of one or more additives; particularly selected from the group consisting of carbonate or hydrogen carbonate of metal or alkaline earth metals such as for example $K_2CO_3$, $KHCO_3$ or $Na_2CO_3$. In an embodiment, step b1) of the process of the invention is performed in the presence of one or more additives in an amount from 1% (w/w) to 10% (w/w).
In an embodiment, step b2) of the process comprises reducing the carbonyl group of the cyclopentanone ring with a reducing agent to yield the cyclopentanol ring containing compound. In an embodiment, step b2) of the process of the invention comprises reducing the carbonyl group of the cyclopentanone ring with a reducing agent selected from the group consisting of alkaline hydride, alkaline borohydride and a mixture thereof. In an embodiment, step b2) of the process of the invention comprises reducing the carbonyl group of the cyclopentanone ring with a reducing agent selected

from the group consisting of $NaBH_4$, $LIBH_4$, $KBH_4$ and a mixture thereof.

In an embodiment, step b2) of the process comprises reducing the carbonyl group of the cyclopentanone ring in the presence of a base. In an embodiment, step b2) of the process comprises reducing the carbonyl group of the cyclopentanone ring in the presence of a base such as for example metal or alkaline earth metal hydroxide. Examples of appropriate hydroxides are NaOH, KOH or LiOH.

In an embodiment, the process of the second aspect of invention further comprises one or more protecting or deprotecting steps. The appropriate protective groups as well as the protecting and deprotecting reaction conditions, and their amounts, can readily be determined by those skilled in the art according to the type of protective group and the structure of the cyclopentanol ring-containing compound.

In an embodiment, the process of the second aspect of the invention is one wherein the cyclopentanol ring-containing compound is a pharmaceutically active ingredient. In an embodiment, the process of the second aspect of the invention is one wherein the cyclopentanol ring-containing compound is a pharmaceutically active ingredient of formula (X)

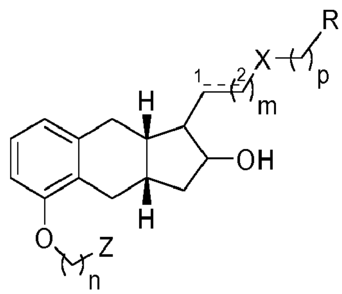

(X)

wherein:

- n is an integer selected from 1, 2 and 3;
- m is an integer selected from 1, 2 and 3;
- p is an integer selected from 0, 1, 2, 3, 4, 5, 6 or 7,
- ----- represents a single bond or a double bond,
- R is selected from the group consisting of $(C_1\text{-}C_6)$alkyl, $(C_5\text{-}C_8)$cycloalkyl, $(C_4\text{-}C_8)$cycloheteroalkyl-, and $(C_1\text{-}C_6)$alkyl-O-;
- X is selected from the group consisting of -C(H)(OH)-, -C(H)(O($C_1$-$C_6$)alkyl) and -C(=O)-,
- Z is selected from the group consisting of -COOH, -CN, -$COOR_4$; and
- R4 is selected from the group consisting of $(C_1\text{-}C_6)$alkyl, phenyl-, phenyl-$CH_2$-.

In an embodiment, the process of the second aspect of the invention is one wherein the cyclopentanol ring-containing compound is a pharmaceutically active ingredient of formula (X) wherein R is selected from the group consisting of $CH_3$-, cyclohexyl-, phenyl-, furyl-, $CH_3O$- and $CH_3$-$CH_2O$-.

In an embodiment, the process of the second aspect of the invention is one wherein the cyclopentanol ring-containing compound is a pharmaceutically active ingredient of formula (X) wherein $R_4$ is selected from the group consisting of $CH_3$-, $CH_3CH_2$-, $(CH_3)_3C$- and phenyl-$CH_2$-.

In an embodiment, the process of the second aspect of the invention is one wherein the cyclopentanol ring-containing compound is the pharmaceutically active ingredient of formula (1)

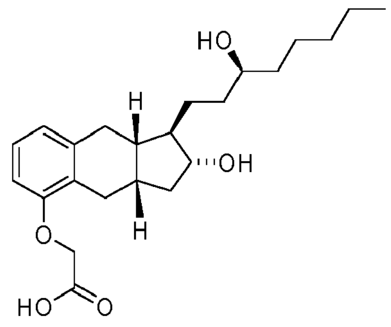

(1)

The compound of formula (1) is also named as treprostinil. Treprostinil is the International Nonproprietary Name (INN) of 1*R*,2*R*,3a*S*,9a*S*)-[[2,3,3a,4,9,9a-Hexahydro-2-hydroxy-1-[(3*S*)-3-hydroxyoctyl]-1*H*-benz[f]inden-5-yl]oxy]acetic acid having the CAS number 81846-19-7.

Treprostinil is a synthetic analogue of prostacyclin (PGI2) and it is a vasodilator agent of arteries in the lungs and body. Treprostinil also inhibits platelet aggregation and smooth muscle proliferation. Treprostinil is indicated for the treatment of pulmonary arterial hypertension. It may be administered as a continuous subcutaneous infusion or continuous intravenous infusion; and it is also available in inhaled and tablet forms. In fact, treprostinil is commercially available under the tradenames Remodulin (for infusion), Orenitram (for oral administration), and Tyvaso (for inhalation).

In an embodiment, the process for the preparation of the cyclopentanol ring-containing compound of formula (1) comprises:

a') performing the continuous flow process as defined in the first aspect of the invention from the alkyne-alkene containing compound of formula (2)

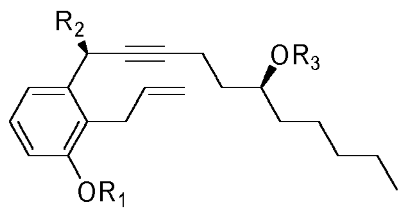

(2)

to yield the cyclopentenone ring-containing compound of formula (3)

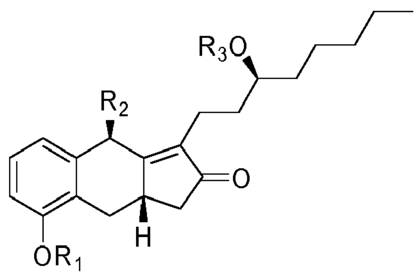

(3)

In an embodiment, the process for the preparation of the cyclopentanol ring-containing compound of formula (1) comprises:

a') performing the continuous flow process as defined in the first aspect of the invention from the alkyne-alkene containing compound of formula (2)

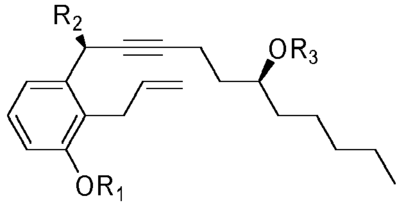

(2)

to yield the cyclopentenone ring-containing compound of formula (3);

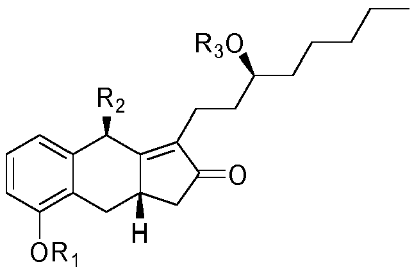

(3)

and

b') submitting the cyclopentenone ring-containing compound of formula (3) under such reaction conditions to convert the cyclopentenone ring-containing compound (3) into the cyclopentanol ring-containing compound (1); wherein:

$R_1$ is PG-O-CO-$CH_2$- or a group convertible into HO-CO-$CH_2$-;
$R_2$ is a HO-, PG-O- or a group convertible into H;
$R_3$ is H- or PG-;
PG is a hydroxy protective group selected from the group consisting of a silane-containing protective group of formula $((C_1\text{-}C_{16})alkyl)_3$-Si-;
and wherein, each one of $R_1$, $R_2$ and $R_3$ is stable under the reaction conditions and is a de-protected form or an intermediate protected form of it that can be transformed after that into said de-protected form in the needed measure.

In an embodiment, the process for the preparation of the cyclopentanol ring-containing compound of formula (1) wherein PG is a silane-containing protective group of formula $((C_1\text{-}C_{16})$alkylh-Si- selected from the group consisting of trimethylsilyl (TMS), *tert*-butyldimethylsilyl (TBDMS), *tert*-butyldiphenylsilyl (TBDPS), triisopropylsilyl (TIPS), triethylsilyl (TES), diethylisopropylsilyl (DEIPS) and isopropyldimethylsilyl (IPDMS).

All the embodiments disclosed above for the Pauson-Khand reaction of the first aspect of the invention also apply to step a') of the process of the second aspect of the invention.

In an embodiment, step (b') of the process for the preparation of cyclopentanol ring-containing compound of formula (1) comprises:

b1') converting the cyclopentenone ring-containing compound of formula (3) into a cyclopentanone ring-containing compound (4)

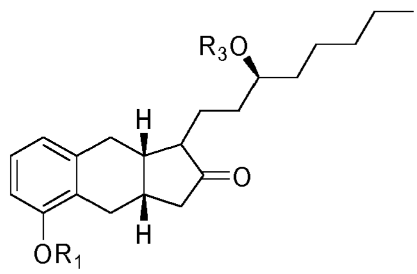

(4)

; and

b2') converting the cyclopentanone ring-containing compound of formula (4) into the cyclopentanol ring-containing compound of formula (1) (treprostinil), wherein $R_1$ and $R_3$ is as defined in the present invention.

All the embodiments disclosed above for steps b1) and b2) also apply to steps b1') and b2') of the process of the second aspect of the invention.

In an embodiment, the process for the preparation of the cyclopentanol ring-containing compound of formula (1) is one wherein:

$R_1$ is PG-O-CO-$CH_2$- and PG is ($C_1$-$C_6$)alkyl-;
$R_2$ is PG-O- and PG is a hydroxy protective group selected form the group consisting of a silane-containing protective group of formula (($C_1$-$C_{16}$)alkyl)$_3$-Si-; and $R_3$ is PG- and PG is a hydroxy protective group selected from the group consisting of a silane-containing protective group of formula (($C_1$-$C_{16}$)alkyl)$_3$-Si-.

In an embodiment, the process for the preparation of the cyclopentanol ring-containing compound of formula (1) is one wherein:

$R_1$ is PG-O-CO-$CH_2$- and PG is ($C_1$-$C_6$)alkyl-;
$R_2$ is PG-O- and PG is a silane-containing protective group of formula (($C_1$-$C_{16}$)alkyl)$_3$-Si-selected from the group consisting of trimethylsilyl (TMS), *tert*-butyldimethylsilyl (TBDMS), *tert*-butyldiphenylsilyl (TBDPS), triisopropylsilyl (TIPS), triethylsilyl (TES), diethylisopropylsilyl (DEIPS) and isopropyldimethylsilyl (IPDMS); and
$R_3$ is PG- and PG is a silane-containing protective group of formula (($C_1$-$C_{16}$)alkyl)$_3$-Si-selected from the group consisting of trimethylsilyl (TMS), *tert*-butyldimethylsilyl (TBDMS), *tert*-butyldiphenylsilyl (TBDPS), triisopropylsilyl (TIPS), triethylsilyl (TES), diethylisopropylsilyl (DEIPS) and isopropyldimethylsilyl (IPDMS).

In an embodiment, the process for the preparation of the cyclopentanol ring-containing compound of formula (1) is one wherein:

$R_1$ is PG-O-CO-$CH_2$- and PG is $(CH_3)_3C$-;
$R_2$ is PG-O and PG is $(CH_3)_3C$-Si(H)-$(CH_3)_2$-; and
$R_3$ is PG- and PG is $(CH_3)C$-Si(phenyl)$_2$-.

It is also a part of the invention a compound selected from the group consisting of:

a compound of formula (2);

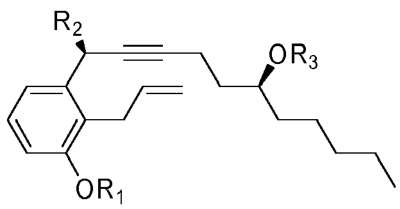

(2)

a compound of formula (3); and

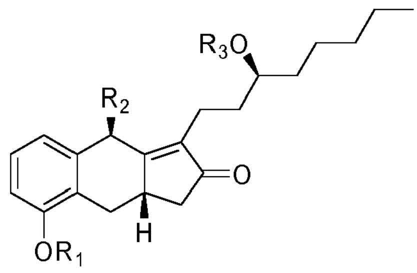

(3)

a compound of formula (4)

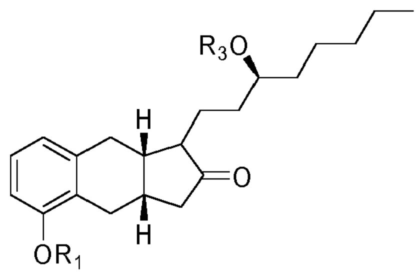

(4)

wherein: $R_1$, $R_2$ and $R_3$ are as defined in the present invention.

In an embodiment, the compound of formula (2) is one wherein:

$R_1$ is $(CH_3)_3C$-O-CO-$CH_2$-;
$R_2$ is $(CH_3)_3C$-Si(H)-$(CH_3)_2$-O; and
$R_3$ is $(CH_3)C$-Si(phenyl)$_2$-.
In an embodiment, the compound of formula (3) is
$R_1$ is $(CH_3)_3C$-O-CO-$CH_2$-;
$R_2$ is $(CH_3)_3C$-Si(H)-$(CH_3)_2$-O; and
$R_3$ is $(CH_3)C$-Si(phenyl)$_2$-.

In an embodiment, the compound of formula (4) is

$R_1$ is $(CH_3)_3C$-O-CO-$CH_2$-;
$R_2$ is H; and
$R_3$ is $(CH_3)C$-Si(phenyl)$_2$-.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

## Examples

### Abbreviations and acronyms

- IPA: isopropanol
- ACN: acetonitrile
- Ts: tosylate
- AcOEt: ethyl acetate
- TBMSO: tertbutylmethylsilyloxy
- TBDPSO: tertbutyldiphenylsilyloxy
- TFA: trifluoroacetic acid
- HPLC: high-performance liquid chromatography
- BPR: back pressure regulator

### General considerations

Solvents used were purchased to VWR (AcOEt, Hexane, ACN, toluene) and Sigma-Aldrich (IPA). For purification of crude reaction mixtures flash chromatography was applied in all cases. Silica-gel (230-400 mesh) was used as the stationary phase (purchased from VWR). The following reagents were purchased from Acros: $Co_2(CO)_8$ and Panreac: HCl, $Na_2SO_4$.

Product 2 was prepared following the process disclosed in the state of the art (García-Lacuna, J. et al., J. Chemistry Conference for Young Scientists (ChemCYS) February 21-23th 2018. N-allyl-4-methyl-N-(prop-2-yn-1-yl)benzenesulfonamide and diethyl 2-allyl-2-(prop-2-yn-1-yl)malonate was prepared following the process disclosed in the state of art (Benedetti, E.; Simonneau, A. et at. L. Adv. Synth. Catal., 2011, vol. 353, pp.1908-1912.

**- HPLC method for the conversion calculation.**

The determination of the conversion of the Pauson-Khand reaction of the present invention is performed using the equipment and the conditions disclosed below:

Equipment and method conditions:
Column: Kromasil C8 5um, 250x4,6 mm
Column temperatura: 30°C
Flow: 1 mL/min
Mobil phase:

- Solvent A: TFA in water 0,1%
- Solvent B: Acetonitrile.

Mobil phase gradient:

| Time | Solvent A | Solvent B |
|---|---|---|
| 0 | 95% | 5% |
| 20 | 5% | 95% |
| 25 | 5% | 95% |

- **HPLC method for the yield calculation**

Different dilutions of the final product were prepared in order to have a representative correlation curve for the final product of Absorbance versus concentration using the following equation: y=1868,7x+57,204 $R^2$=0,9951:

| Concentration (μmol/mL)) | Area (Absorbance units) |
|---|---|
| 5,716 | 10951,6 |

(continued)

| Concentration (μmol/mL)) | Area (Absorbance units) |
|---|---|
| 2,858 | 4921 |
| 0,8575 | 1786,5 |
| 0,2858 | 728,9 |

Equipment and method conditions:

Column: Kromasil C8 5um, 250x4,6 mm
Column temperatura: 30°C
Flow: 1 mL/min
Mobil phase:

Solvent A: TFA in water 0,1%
Solvent B: Acetonitrile

Mobil phase gradient:

| Time | Solvent A | Solvent B |
|---|---|---|
| 0 | 95% | 5% |
| 20 | 5% | 95% |
| 25 | 5% | 95% |

**Example 1. Continuous Flow Synthesis of 2-tosyl-2,3,3a,4-tetrahydrocyclopenta[c]pyrrol-5(1H)-one**

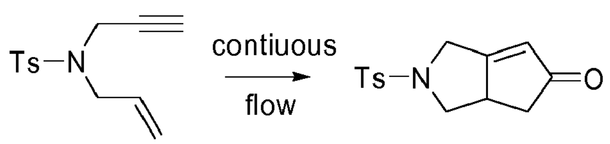


**Scheme 2**

The Plug Flow Reactor system of the reaction of Scheme 2 is configured as shown in Figure 1. It consists of a HPLC pump that continuously provides the solvent, a Rheodyne valve which introduces the diluted substrate, a 60.6mL stainless steel reactor, which is inside an oven, and a back-pressure regulator (BPR):

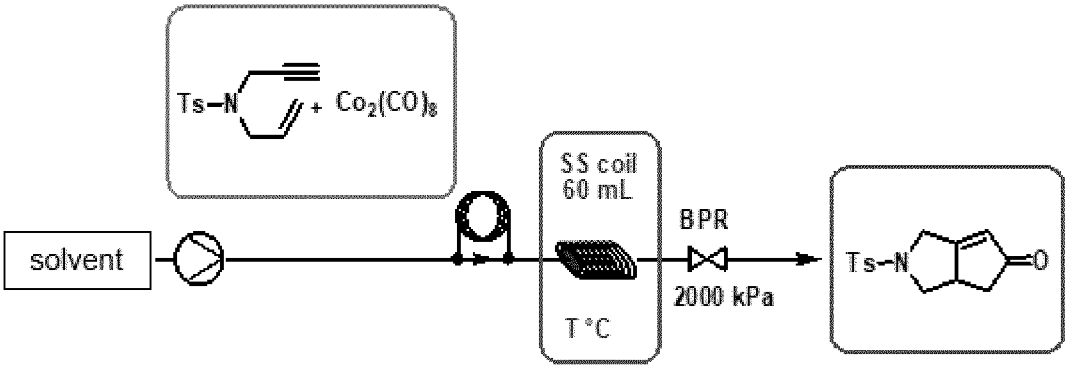


Figure 1

A 2 mL stock solution of the substrate is prepared each time just before injections. 199.5 mg (0.8 mmol) of N-allyl-4-methyl-N-(prop-2-yn-1-yl)benzenesulfonamide were added to a solution of 1 mL of 41 mg of $Co_2(CO)_8$(15 mol%) in the solvent indicated in each entry (Table 1). Then, the solvent was added until a final volume of 2 mL. 0.8 mL were injected each time through the Rheodyne valve into the oven at the temperature indicated in each entry (Table 1) with the pressure of the system fixed at 2000 kPa. Samples were collected after the residence time selected in each case in test tubes, 2 mL of the collected solution were evaporated in vacuo and after, dissolved in 2 mL of HPLC ACN. The obtained sample were filtered through a 0.45 um filter and it was analysed by HPLC using the method disclosed in the general consideration section.

The resulting mixture were washed with 1M HCl solution (3 mL), brine (3mL), dried over $Na_2SO_4$ and the solvent evaporated *in vacuo*. Then, silica gel column chromatography in Hexane:Ethyl acetate (3:1) gave pure 2-tosyl-2,3,3a,4-tetrahydrocyclopenta[c]pyrrol-5(1H)-one as a white solid.

$^1$H-NMR (400 MHz, $CDCl_3$) $\delta$ = 7.66 (d, *J* = 8.2, 2H, ArH); 7.29 (d, *J* = 8.1, 2H, ArH), 5.92 (s, 1H, HC=), 4.27 (d, *J* = 16.5, 1H, NCHHCH), 4.00 - 3.93 (dd, *J* = 14.3, 5.1, 2H, NCHHCH and NCHHC=), 3.04 - 3.10 (m, 1H, CH), 2.60 - 2.48 (m, 2H, CHHCO and NCHHC=), 2.37 (s, 3H, $CH_3$), 2.03 - 1.96 (dd, $J_1$ = 17.9, $J_2$= 3.7, 1H, CHHCO) ppm.

**Table 1**

| Entry | Temp. (°C) | Pressure (kPa) | Mol% $Co_2(CO)_8$ | Solvent | Rt (b) | Conversion (%) | Yield (%) HPLC | Yield (isolated) |
|---|---|---|---|---|---|---|---|---|
| **1** | 110 | 2000 | 15 | toluene | 35 min | 91 | 65 | - |
| **2** | 110 | 2000 | 15 | toluene | 44 min | 95 | 70 | 65 |
| **3** | 110 | 2000 | 40 | toluene | 44 min | >99 | 73 | 66 |
| **4** | 130 | 2000 | 15 | toluene | 22 min | 97 | 67 | - |
| **5** | 130 | 2000 | 15 | toluene | 36 min | >99 | 72 | 63 |
| **Comp. 6** | 110 | 2000 | 15 | toluene | 18 hours | 90 | 35 | 25 |

(a) Comparative Example 2 batch reaction falling outside the scope of the invention
(b) Rt: residence time which corresponds to the reaction time measured in minutes

**Comparative Example 2. Batch Synthesis of 2-tosyl-2,3,3a,4-tetrahydrocyclopenta[c]pyrrol-5(1H)-one**

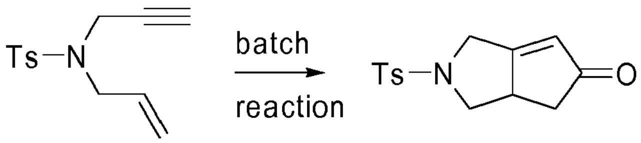

199.5 mg (0.8 mmol) of the starting N-allyl-4-methyl-N-(prop-2-yn-1-yl)benzenesulfonamide were added to a solution of 2 mL of 41 mg of $Co_2(CO)_8$ (15 mol%) in toluene in a stainless-steel tube. The tube was purged with $N_2$ tree times and then pressurized to achieve 2000 kPa when heated to 110°C. The mixture was stirred for 18 h. The crude reaction was filtered through zeolite and solvent was then *removed in vacuo.* 1 mg of the crude was dissolved in 2 mL ACN and that sample is analysed by HPLC (using the method disclosed in the general consideration section) giving a 35% yield. After washing and purification by column chromatography following the process disclosed in Example 1 procedure 25% of pure cyclopentenone ring-containing compound was recovered shown in comparative entry 6 of Table 1 above.

**Example 3. Continuous flow Synthesis of diethyl 5-oxo-3,3a,4,5-tetrahydropentalene-2,2(1 H)-dicarboxylate**

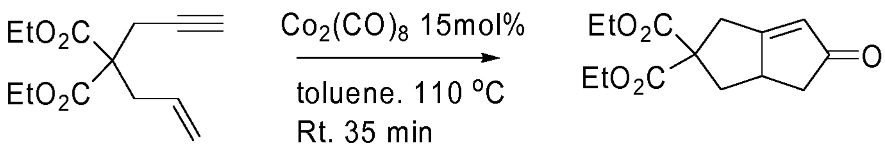


A 2 mL solution of diethyl 2-allyl-2-(prop-2-yn-1-yl)malonate was prepared. 190.6 mg (0.8 mmol) of diethyl 2-allyl-2-(prop-2-yn-1-yl)malonate were added to a solution of 1 mL of 41 mg of $Co_2(CO)_8$ (15 mol%) in toluene. Then, toluene was added until a final volume of 2 mL. 0.8 mL were injected through the Rheodyne valve. Temperature was set at 110 °C, pressure at 2000 kPa and residence time of 35 min. Crude reaction was collected in a test tube, washed with 1M HCl, solution (3 mL), brine (3mL), dried with $Na_2SO_4$ and solvent evaporated *in vacuo.* Then, the resulting compound was purified through a silica gel column chromatography in Hexane:Ethyl acetate (9:1) to afford 59 mg (69% yield) as a colorless oil of diethyl 5-oxo-3,3a,4,5-tetrahydropentalene-2,2(1H)-dicarboxylate.

$^1$H-NMR (400 MHz, $CDCl_3$): 5.89 (s, 1H, C=CH), 4.24 - 4.13 (m, 4H, $2xCH_2O$), 3.35 - 3.18 (m, 2H), 3.14 - 3.00 (in,1H1), 2.76 (dd, *J*= 12.7 and 7.6 Hz), 2.59 (dd, *J*= 17.9 and 6.4 Hz, 1H), 2.09 (dd, *J*= 17.8 and 3.2 Hz, 1H), 1.70 (t, *J*= 12.7 Hz, 1H), 1.21 (q, *J*= 7.3 Hz, 6H, $2xCH_3$).

**Example 4. Synthesis of tert-butyl 2-(((4R,9aS)-4-((tert-butyldimethylsilyl)oxy)-3-((S)-3-((tert-butyldiphenylsilyl)oxy)octyl)-2-oxo-2,4,9,9a-tetrahydro-1H-cyclopenta[b]naphthalen-8-yl)oxy)acetate**

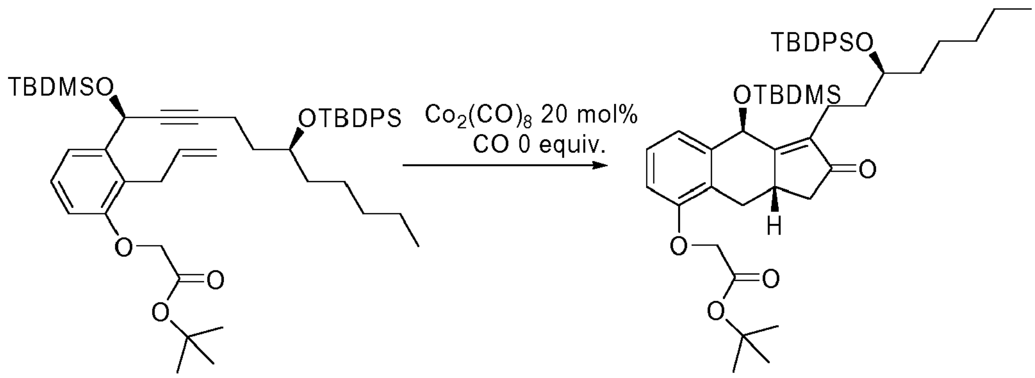


A 2 mL stock solution of *tert-butyl* 2-(2-allyl-3-((5*S*,10*S*)-2,2,3,3,13,13-hexamethyl-10-pentyl-12,12-diphenyl-,11-dioxa-3,12-disilatetradec-6-yn-5-yl)phenoxy)acetate was prepared each time just before injections. 469 mg (0.6 mmol) of 21 were added to a solution of 1 mL of 30.8 mg or 41.0 mg of $Co_2(CO)_8$ in the mol% indicated in each entry (Table 2) in toluene. Then, toluene was added until a final volume of 2 mL. 0.8 mL were injected each time through the Rheodyne valve into the oven at the temperature indicated in each entry (Table 2) with the pressure of the system fixed at 2000 kPa. Crude reaction was collected in a test tube and analysed by HPLC using the method disclosed in the general consideration section.

The resulting mixture were washed with 1M HCl solution (3 mL), brine (3mL), dried with $Na_2SO_4$ and solvent evaporated *in vacuo.* Then, silica gel column chromatography in Hexane:Ethyl acetate (12:1) gave 22 as a yellow oil.

Retardation factor (Rf): 0.43 (Hexane/AcOEt 9:1)

[α]$^{25}_{D}$ -87.4 (c 0.42, $CHCl_3$)
IR (NaCl) ν = 2956, 2936, 2958, 2754, 1754, 1707, 1664, 1589, and 1475 $cm^{-1}$.

Elemental analysis calcd for $C_{49}H_{70}O_6Si_2$: C, 73.61; H, 9.01; Si, 7.17 found: C 73.72, H 9.13. $^1H$ NMR (400 MHz, $CDCl_3$) δ 7.64 - 7.59 (m, 4H, Ar), 7.42 - 7.25 (m, 6H), 7.19 (t, *J* = 7.9 Hz, 1H, Ar), 6.87 (d, *J* = 7.6 Hz, 1H, Ar), 6.66 (d, *J* = 7.7 Hz, 1H, Ar), 5.47 (s, 1H, C*H*OTBS), 4.54 (s, 2H, $CH_2O$), 3.75 - 3.71 (m, 1H, CHOSi), 3.62 (dd, $J_1$ = 17.2 Hz, $J_2$ = 7.5 Hz, 1H, C*H*HAr), 3.37 - 3.30 (m, 1H, C*H*), 2.67 (dd, $J_1$ = 18.8 Hz, $J_2$ = 6.4 Hz, 1H, C*H*HCO), 2.44 - 2.34 (m, 1H, C*H*HAr), 2.29 - 2.19 (m, 2H, C*H*HCO and C*H*HC=), 1.68 - 1.59 (m, 2H, C*H*HC= and C*H*H), 1.48 (s, 9H, $H_3C)_3CO$), 1.44-1.36 (m, 3H, $CH_2$ and C*H*H), 1.18 - 1.11 (m, 6H, $3xCH_2$), 1.06 (s, 9H, $(H_3C)_3CSi$), 0.81 (s, 9H, $(H_3C)_3CSi$), 0.78 (t, *J* = 7.2 Hz, 3H, $CH_3$), 0.10 (s, 3H, $H_3CSi$), 0.05 (s, 3H, $H_3CSi$) ppm.

**Table 2**

| Entry | Temp. (°C) | Pressure (kPa) | Mol % $Co_2(CO)_8$ | Rt (min) | Conversion (%) | Yield HPLC (%) | Yield isolated (%) |
|---|---|---|---|---|---|---|---|
| 2 | 170 | 2000 | 15 | 40 | 95 | 59 | - |
| 3 | 170 | 2000 | 30 | 40 | >99 | 73 | - |
| 4 | 170 | 2000 | 20 | 40 | >99 | 68 | - |
| 5 | 170 | 2000 | 20 | 30 | >99 | 72 | 65 |
| 6 | 200 | 2000 | 20 | 30 | >99 | 58 | 45 |
| 7[a] | 170 | 2000 | 20 | 30 | >99 | 64 | 58 |
| (a) The reaction was performed following the process as defined in Entry 5 but using 1g of compound 21 | | | | | | | |

## Claims

1. A continuous flow process for the preparation of a cyclopentenone ring-containing compound which comprises:

   reacting an alkyne-moiety with an alkene-moiety
   in the presence of a catalytically effective amount of one or more metal carbonyl compounds,
   in the absence of exogenous carbon monoxide gas;

   wherein:

   when the process is an intermolecular process, the process comprises reacting the alkyne-moiety of an alkyne-containing compound with the alkene-moiety of an alkene-containing compound; and
   when the process is an intramolecular process, the process comprises reacting the alkyne-moiety with the alkene-moiety of an alkyne-alkene containing compound.

2. The continuous flow process according to claim 1, wherein the metal of the metal carbonyl compound is selected from the group consisting of cobalt, rhodium, iridium, tungsten, molybdenum, titanium, nickel, iron and ruthenium; preferably cobalt.

3. The continuous flow process according to any of the claims 1 or 2, wherein the metal carbonyl compound is dicobalt octacarbonyl.

4. The continuous flow process according to any of the claims 1-3, wherein the catalytically effective amount of the metal carbonyl compound is from 15 to 40 mol% of the alkyne-moiety containing compound or alternatively of the alkyne-alkene containing compound.

5. The continuous flow process according to any of the claims 1-4, which has a reaction conversion equal to or higher than 90% and a chemical yield of the cyclopentenone ring-containing compound equal to or higher than 60%.

6. The continuous flow process according to any of the claims 1-5, which is performed at a temperature from 100°C to 180°C and at a pressure from 1000 kPa to 3000 kPa.

**7.** The continuous flow process according to any of the claims 1-6, wherein the reaction is performed in the presence of an organic solvent selected from the group consisting of toluene, xylene, naphthalene, isopropanol, ethanol, tert-butanol, tetrahydrofuran, 2-methyltetrahydrofuran, dioxane and a mixture thereof.

**8.** The continuous flow process according to any of the claims 1-7, wherein:

the process is an intramolecular process and the alkyne-alkene containing compound is a compound of formula (2)

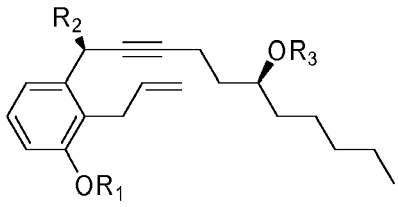

(2)

and the cyclopentenone ring-containing compound is a compound of formula (3)

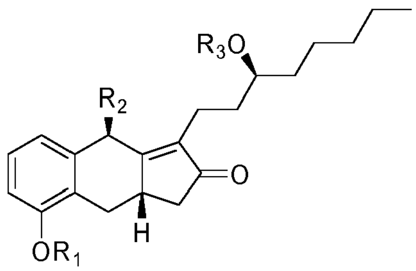

(3)

wherein:

$R_1$ is PG-O-CO-$CH_2$- or a group convertible into HO-CO-$CH_2$-;
$R_2$ is a HO-, PG-O- or a group convertible into H;
$R_3$ is H- or PG-;
PG is a hydroxy protective group selected from a silane-containing protective group of formula $((C_1\text{-}C_{16})alkyl)_3$-Si-;

and wherein, each one of $R_1$, $R_2$ and $R_3$ is stable under the reaction conditions and is a de-protected form or an intermediate protected form of it that can be transformed after that into said de-protected form in the needed measure.

**9.** A process for the preparation of cyclopentanol ring-containing compound which comprises:

a) performing the continuous flow process as defined in any of the claims 1-8 to yield a cyclopentenone ring-containing compound; and
b) submitting the cyclopentenone ring-containing compound under such reaction conditions to convert the cyclopentenone ring-containing compound into the cyclopentanol ring-containing compound.

**10.** The process according to claim 9, wherein the cyclopentanol ring-containing compound is a pharmaceutically active ingredient of formula (X)

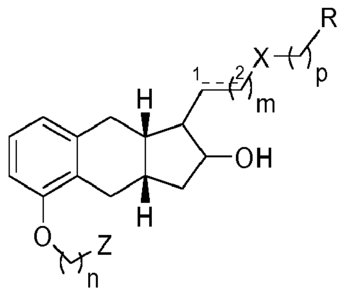

(X)

wherein:

n is an integer selected from 1, 2 and 3;
m is an integer selected from 1, 2 and 3;
p is an integer selected from 0, 1, 2, 3, 4, 5, 6 or 7,
----- represents a single bond or a double bond,
R is selected from the group consisting of $(C_1-C_6)$alkyl, $(C_5-C_8)$cycloalkyl, $(C_4-C_8)$cycloheteroalkyl-, and $(C_1-C_6)$alkyl-O-;
X is selected from the group consisting of -C(H)(OH)-, -C(H)(O$(C_1-C_6)$alkyl) and -C(=O)-,
Z is selected from the group consisting of -COOH, -CN, $-COOR_4$; and
R4 is selected from the group consisting of $(C_1-C_6)$alkyl, phenyl-, phenyl-$CH_2$-.

**11.** The process according to claim 10, wherein the cyclopentanol ring-containing compound (X) is a compound of formula (1)

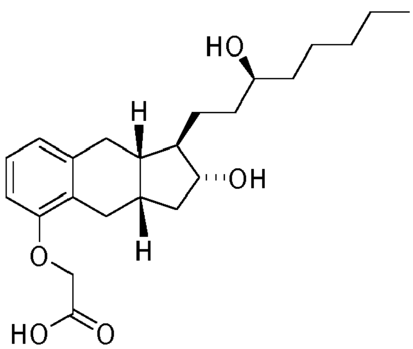

(1)

**12.** The process according to claim 11, wherein the process comprises:

a') submitting the compound of formula (2)

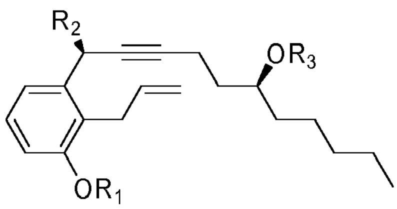

(2)

under the reaction conditions defined in any of the claims 1-8 to obtain the cyclopentenone ring-containing compound of formula (3)

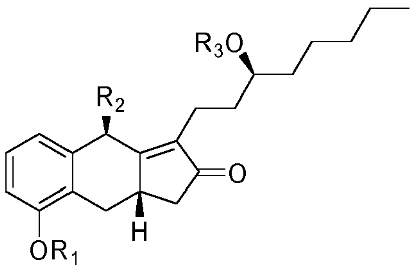

(3)

wherein:

$R_1$ is PG-O-CO-$CH_2$- or a group convertible into HO-CO-$CH_2$-;
$R_2$ is a HO-, PG-O- or a group convertible into H;
$R_3$ is H- or PG-;
PG is a hydroxy protective group selected from the group consisting of a silane-containing protective group of formula (($C_1$-$C_{16}$)alkyl)$_3$-Si-; and
wherein, each one of $R_1$, $R_2$ and $R_3$ is stable under the reaction conditions and is a de-protected form or an intermediate protected form of it that can be transformed after that into said de-protected form in the needed measure.

**13.** The process according to any of the claims 11 or 12, wherein the process of converting the cyclopentenone ring-containing compound of formula (3) into the cyclopentanol ring-containing compound of formula (1) comprises:

b1') converting the compound of formula (3) into a cyclopentanone ring-containing compound of formula (4)

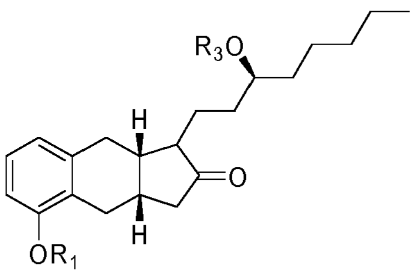

(4)

and
b2') converting the cyclopentanone ring-containing compound of formula (4) into the cyclopentanol ring-containing compound of formula (1), wherein $R_1$ and $R_3$ is as defined in claim 12.

**14.** The process according to any of the claims 11-13, wherein:

$R_1$ is PG-O-CO-$CH_2$- and PG is ($C_1$-$C_6$) alkyl-;
$R_2$ is PG-O- and PG is a silane-containing protective group of formula (($C_1$-$C_{16}$)alkyl)$_3$-Si-selected from the group consisting of trimethylsilyl, *tert*-butyldimethylsilyl, *tert*-butyldiphenylsilyl, triisopropylsilyl, triethylsilyl, diethylisopropylsilyl and isopropyldimethylsilyl; and
$R_3$ is PG- and PG is a silane-containing protective group of formula (($C_1$-$C_{16}$)alkyl)$_3$-Si-selected from the group consisting of trimethylsilyl, *tert*-butyldimethylsilyl, *tert*-butyldiphenylsilyl, triisopropylsilyl, triethylsilyl, diethylisopropylsilyl and isopropyldimethylsilyl.

**15.** The process according to any of the claims 11-14, wherein

$R_1$ is PG-O-CO-$CH_2$- and PG is $(CH_3)_3C$-;

$R_2$ is PG-O and PG is $(CH_3)_3C-Si(H)-(CH_3)_2-$; and
$R_3$ is PG- and PG is $(CH_3)C-Si(phenyl)_2-$.

**16.** A compound selected from the group consisting of:

a compound of formula (2);

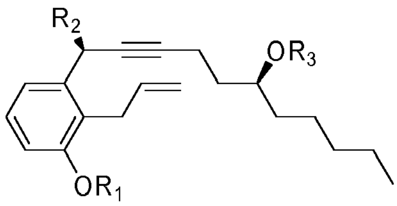

(2)

a compound of formula (3); and

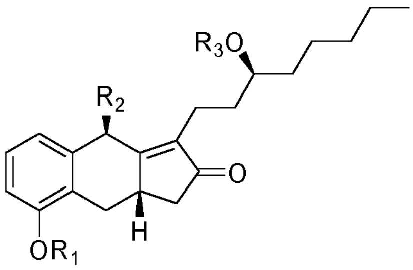

(3)

a compound of formula (4)

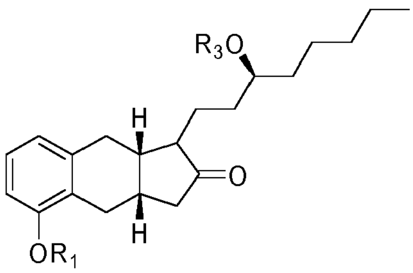

(4)

wherein: $R_1$, $R_2$ and $R_3$ is as defined in any of the claims 1-15.

Europäisches Patentamt
European Patent Office
Office européen des brevets

# EUROPEAN SEARCH REPORT

Application Number
EP 19 38 2748

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JORGE GARCÍA-LACUNA ET AL: "A catalytic scalable Pauson-Khand reaction in a plug flow reactor", CHEMICAL COMMUNICATIONS, vol. 53, no. 28, 1 January 2017 (2017-01-01), pages 4014-4017, XP055668849, UK ISSN: 1359-7345, DOI: 10.1039/C7CC01749A * table 1 * ----- | 1-15 | INV. C07C45/49 C07C49/597 C07C69/757 C07F7/08 |
| Y | US 2009/306403 A1 (PERICAS-BRONDO MIGUEL ANGEL [ES] ET AL) 10 December 2009 (2009-12-10) * paragraph [0059] * ----- | 1-15 | |
| Y | KEISUKE ASANO ET AL: "Pauson-Khand Reactions in a Photochemical Flow Microreactor", ORGANIC LETTERS, vol. 15, no. 10, 17 May 2013 (2013-05-17), pages 2398-2401, XP055668981, ISSN: 1523-7060, DOI: 10.1021/ol4008519 * tables 2,3 * ----- | 1-15 | TECHNICAL FIELDS SEARCHED (IPC): C07C C07F |
| A | WO 2012/009816 A1 (ALPHORA RES INC [CA]; MCGOWAN GRAHAM [CA] ET AL.) 26 January 2012 (2012-01-26) * claim 7; figure 1/1 * ----- | 1-15 | |

1 ~~The present search report has been drawn up for all claims~~

| Place of search | Date of completion of the search | Examiner |
|---|---|---|
| The Hague | 17 February 2020 | Matés Valdivielso, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 19 38 2748

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 19 38 2748

The Search Division considers that the present European patent application does not comply with the requirements of unity of invention and relates to several inventions or groups of inventions, namely:

1. claims: 1-15

   A continuous flow process for the preparation of a cyclopentenone ring-containing compound

   ---

2. claim: 16

   Compounds of formulae (2), (3) and (4)

   ---

## ANNEX TO THE EUROPEAN SEARCH REPORT ON EUROPEAN PATENT APPLICATION NO.

EP 19 38 2748

This annex lists the patent family members relating to the patent documents cited in the above-mentioned European search report. The members are as contained in the European Patent Office EDP file on The European Patent Office is in no way liable for these particulars which are merely given for the purpose of information.

17-02-2020

| Patent document cited in search report | Publication date | Patent family member(s) | Publication date |
|---|---|---|---|
| US 2009306403 A1 | 10-12-2009 | CA 2641405 A1 | 15-11-2007 |
| | | CN 101432266 A | 13-05-2009 |
| | | EP 1849770 A1 | 31-10-2007 |
| | | EP 2076493 A1 | 08-07-2009 |
| | | JP 2009535314 A | 01-10-2009 |
| | | US 2009306403 A1 | 10-12-2009 |
| | | WO 2007128457 A1 | 15-11-2007 |
| WO 2012009816 A1 | 26-01-2012 | CA 2710726 A1 | 22-01-2012 |
| | | US 2013331593 A1 | 12-12-2013 |
| | | WO 2012009816 A1 | 26-01-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **T. W. GREEN ; P. G. M. WUTS.** Protective Groups in Organic Chemistry. Wiley, 1999, 17-200
- *CHEMICAL ABSTRACTS,* 81846-19-7
- **GARCÍA-LACUNA, J. et al.** *J. Chemistry Conference for Young Scientists (ChemCYS),* 21 February 2018
- **BENEDETTI, E. ; SIMONNEAU, A.** *L. Adv. Synth. Catal.,* 2011, vol. 353, 1908-1912